# EUROPEAN PATENT SPECIFICATION

(11) **EP 3 174 479 B1**
(45) Date of publication and mention of the grant of the patent: **13.11.2019**
(21) Application number: 15763650.7
(22) Date of filing: 24.06.2015
(51) Int. Cl.: A61B 17/42, A61B 17/3203, A61M 3/02

(54) **PORTABLE SYSTEM FOR HEATING, WASHING AND SUCKING LIQUIDS FOR LAPAROSCOPIC AND LAPAROTOMIC SURGERY, GYNECOLOGY AND UROLOGY**
TRAGBARES SYSTEM ZUM ERWÄRMEN, WASCHEN UND ABSAUGEN VON FLÜSSIGKEITEN FÜR DIE LAPROSKOPISCHE UND LAPAROTOME CHIRURGIE, UROLOGIE UND GYNÄKOLOGIE
SYSTÈME PORTABLE POUR CHAUFFER, LAVER ET ASPIRER DES LIQUIDES POUR LA CHIRURGIE LAPAROSCOPIQUE ET LAPAROTOMIQUE, LA GYNÉCOLOGIE ET L'UROLOGIE

(30) Priority: 28.07.2014 IT TO20140599
(43) Date of publication of application: 07.06.2017
(73) Proprietor: ABC Medical S.r.l., 10144 Torino (IT)
(72) Inventor: POSSEKEL, Roberto, I-10144 Torino (IT)
(74) Representative: Garavelli, Paolo
(86) International application number: PCT/IT2015/000168
(87) International publication number: WO 2016/016910

(56) References cited:
- CN-U- 203 220 631
- RO-A2- 126 083
- US-A- 4 678 460
- US-A- 5 178 606
- US-A- 5 419 772
- US-A- 6 159 160
- US-A1- 2009 309 466
- US-A1- 2010 256 562
- US-B1- 6 748 164

## Description

The present invention refers to a portable system for heating, washing and sucking liquids for laparoscopic and laparotomic surgery, gynecology and urology.

The system of the invention stems from the need of using washing liquids at controlled temperature (from ambient to 40 °C maximum) and outlet flow in laparoscopic and laparotomic surgery, gynecology and urology. In these fields, the operators have the need of using washing systems for cleaning the surgery field from hemorrhages, coagulations or biologic liquids produced by a human organism.

In the majority of operating rooms, the washing system is simply composed of a bag with saline solution connected to a tube that conveys the washing liquid to a cannula due to a fall. The solution is used at ambient temperature (20 - 22 °C) or heated in microwave ovens and bag-heating devices: in both cases, the temperature of the physiologic liquid is not controlled, but dangerously boiling.

In some cases, peristaltic pumps with limited regulation are used, equipped with dedicated and costly disposable circuits.

As regards suction, another disposable circuit is used, connected to the room sucking device. US 5 178 606 A discloses a system for heating, washing and sucking liquids for laparoscopic and laparotomic surgery, gynaecology and urology, comprising: a tank for liquids, at least one peristaltic pump for producing a variable jet of liquid inside an abdominal recess, and at least one valve for sucking liquids in order to wash the surgery field; and at least one heating device for keeping the temperature of the liquids at an established maximum value, heating the tank and a container.

These methodologies imply the continuous presence of nurse personnel, who must guarantee assistance to the operation of the various above listed components.

The system of the invention, instead, guarantees a physiologic solution at a temperature and outlet flow which can be set by a suitable display and can be verified at any time during the intervention. The nurse room personnel, once having started the system, will then be able to perform all other necessary jobs for the correct maintenance of a surgery room.

The system of the invention has therein three functionalities in a single apparatus:
1. heating of the washing liquid
2. washing
3. sucking.

As regards heating of the washing liquid, as pointed out above, risks of burns occur for the nurse personnel and for the patient's internal organs, since the operator is never made aware of the temperature of the washing liquid which, at the beginning of the treatment, is boiling, but after some minutes goes back to ambient temperature: the responsible personnel must therefore concentrate their attention on the washing needs of the operator and, given the short time of maintenance of the temperature, this pre-heating is completely useless.

In the inventive system, instead, the bags of physiologic solution are immersed in a heated tank which can be set by a display at the established temperature, preferably up to a maximum of 40 °C. Such temperature is kept and monitored through the display itself during the whole intervention, while the responsible personnel will only have to replace the bag when it is emptied. This does not imply any risk or problem, and the operator has a hot solution available at any time during the intervention.

As regards washing, in the majority of cases it occurs due to gravity; in some cases, a peristaltic pump is used, often with a difficult regulation, not immediate and not accurate. The consequent risks are the impossibility of quickly washing with efficiency, and in any case without an accurate regulation, or with a wholly absent regulation, of the washing flow. In case of hemorrhages of a medium entity, it is necessary to convert the intervention from laparoscopy to laparotomy, with risks and damages for the patient and a double expense of materials for a single intervention. The counterindications are that washing by gravity does not allow removing clots from the surgery field, does not allow performing the hydro-dissection in adhesiolysis episodes, and therefore results slow and scarcely efficient: in conclusion, the scarce accuracy and the complexity of managing a peristaltic pump or washing by gravity do not enable regularity and stability for intervention phases.

In the inventive system, instead, together with the heating tank, a peristaltic pump is installed. The flow regulation is simple and immediate through the display; it can occur from a minimum of 200 ml/min (ideal for laparotomy) to a maximum of 1950 ml/min (ideal for hydrodissection). This does not imply risks or problems, and an easy management and a quick regulation change of the flow provide the operator with the safety of having a system which can be quickly suited to the type of procedure during the whole intervention.

As regards sucking, currently, in parallel with the disposable set for washing, a second separate set for sucking is prepared, connected to the central sucking system of the room, and therefore with continuous operation. In some cases, cannulas are used of the "flute" type, with valve opening keys which allow the saline solution to go out. This implies the risk that, since the sucking flow is continuous and cannot be managed by the operator, if soft tissues are next to the cannula, they are sucked, clogging the cannula itself. The flute cannulas often are inhibited in their function for forming clots around the valves which do not allow any more their opening/closing. As counterindications, often the operator must extract the sucking cannula for restoring the operation, while, in case of occlusion from a clot, the disposable device must be replaced with another new device with related increase of costs: in conclusion, currently, this solution is scarcely safe for the operator and scarcely efficient in checking the sucking function.

In the inventive system, instead, sucking is regulated by a gate-type valve, which can be easily controlled with a suitable pedal. The sucking flow is not continuous and the operator can manage the various steps depending on the needs dictated by the intervention. When the valve is closed, a depression is created, which amplifies the suction effect upon the first pressure of the pedal, allowing to suck also big clots. The disposable set is single for washing and sucking, while the pedal commands guarantee the lack of occlusion of the cannula or of the set. There are no risks or problems, and there is a total control safety by the operator and a great sucking efficiency; the chamfered cannula for multiple uses made of stainless steel allows its use as palpating tool.

As described above in detail, therefore, object of the present invention is solving the above prior art problems, by providing a portable system for heating, washing and sucking liquids for laparoscopic and laparotomic surgery, gynecology and urology, which is adapted to be transported, compact, easy to use and at the same time equipped with all required functionalities for this type of hospital operations.

The above and other objects and advantages of the invention, as will result from the following description, are obtained with a portable system for heating, washing and sucking liquids for laparoscopic and laparotomic surgery, gynecology and urology as claimed in claim 1. Preferred embodiment and non-trivial variations of the present invention are the subject matter of the dependent claims.

It is intended that all enclosed claims are an integral part of the present description.

The present invention will be better described by some preferred embodiments thereof, provided as a non-limiting example, with reference to the enclosed drawings, in which:

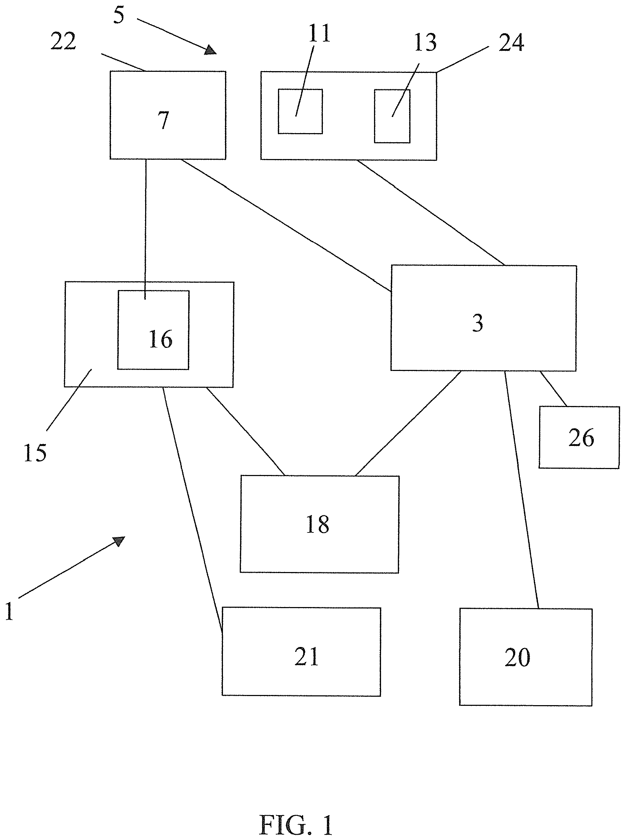

- Figure 1 is a schematic view of a preferred embodiment of the system according to the present invention, in which the component parts are designated with references to blocks;

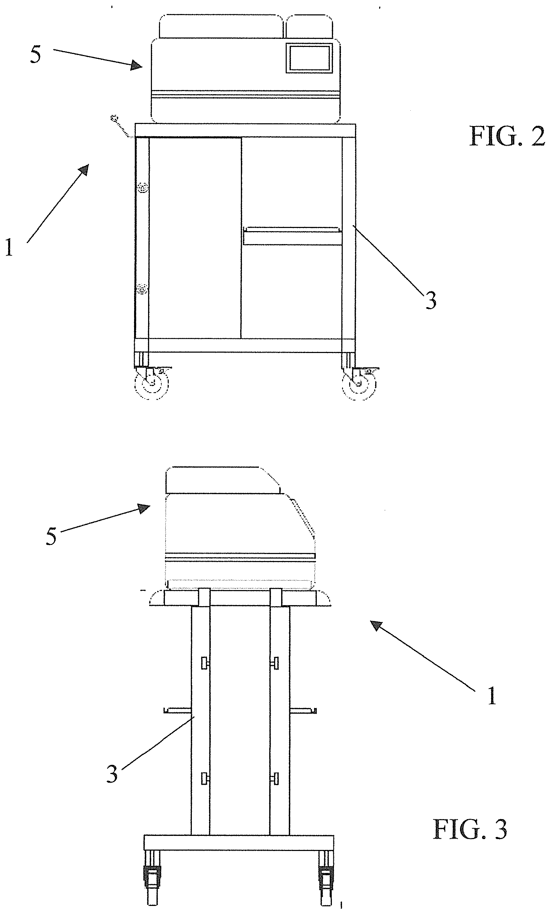

- Figure 2 is a front schematic view of a preferred embodiment of the system according to the present invention; and
- Figure 3 is a side view of the system of Figure 2.

With reference to the Figures, a preferred embodiment of the system of the present invention is shown and described.

It will be immediately obvious that numerous variations and modifications (for example related to shape, sizes, arrangements and parts with equivalent functionality) could be made to what is described, without departing from the scope of the invention, as will appear from the enclosed claims.

According to the preferred, but not limiting, embodiment as shown, the portable system 1 for heating, washing and sucking liquids (preferably heated sterile physiologic water) for laparoscopic and laparotomic surgery, gynecology and urology according to the present invention comprises:
- at least one trolley 3 for supporting and handling;
- at least one case 5 placed on the trolley 3 and composed of: a first part made as a tank 7 for liquids equipped with an inclined bottom 9 to facilitate the discharge of liquids and the cleaning of the tank 7; and a second part containing at least one peristaltic pump 11 for making a variable jet of liquid inside abdominal cavities, and at least one valve 13 to suck liquids for washing the surgery field;
- at least one container 15 of liquids placed on the trolley 3 and equipped with a double pump 16 for taking and removing liquids into and from the tank 7; and
- at least one device 18 for keeping the temperature of liquids at an established maximum value, preferably 40 °C.

The presence of the double pump 16 in the container 15 of liquids allows saving a lot of time in surgery rooms, enables the use of the inventive system 1 and allows always supplying clean water, free from limestone.

The inventive system 1 can be further equipped with a displaying device 20 for setting the operating parameters of the system 1 itself. In particular, such displaying device 20 is of the touch-screen type and allows entering operating parameters and commands for the system 1.

Moreover, the inventive system 1 can be equipped with at least one depurating device 21 for liquids in order to keep them clean upon every loading and emptying passage of the tank 7.

For a better operation, the case 5 is equipped with two separate covers 22, 24 for the two parts of which it is composed, such covers 22, 24 providing the chance of having different opening widths, with room for the passage of disposable tubes for liquids, in order to avoid that tubes are squashed by possible manouvres.

In particular, the trolley 3 can be equipped with a plurality, preferably four, of pivoting wheels 26 which can be blocked, for its operating handling.

Still in particular, the valve 13 for sucking liquids can be of the gate type and is controlled by control means, preferably of the pedal type.

Finally, the system 1 can also be equipped with a blocking device for the case 5 to prevent it from possibly falling.

With the inventive system 1, it is thereby possible to use hot liquids, in particular water for this type of application, with the following clinical advantages:
1) Compression: the saline solution quickly washes the surgery field and dilutes the clots, allowing the sucking system to also remove the biggest clots. It also reduces the concentration of peritoneal liquids, the formation of displasies and protects the peritoneum from lesions. Moreover, compressed hot water helps keeping the optics clean.
2) Thermo-hemostasis: the use of a saline solution at 40 °C during the whole intervention removes the risks of hypothermia and accelerates biologic hemostases.
3) Hydrodissection: the outlet pressuse of the saline solution between 0 and 1.5 bar allows separating the tissues in adhesiolysis episodes.
4) Hydroprotection: in destruction procedures of peritoneal pathologies, such as laser, CO2 or bipolar electro-coagulation, the saline solution can protect the structures below, like pelvic cavity, urether, prosthate and rectum vessels.
5) Hydro-floatation: in gynecology, due to fertility problems of the Douglas, a sub-aquatic inspection is necessary, and in any type of surgery the sub-aquatic inspection of the surgery field is very useful, in modo topoint out possible hemorrhages.
6) Waking up: in cooperation with anesthesiologists, from 100 to 300 cc of saline solution are left in the peritoneal cavity: this allows a quick rehidration of the peritoneum, replaces and complements the intravenous perfusion.
7) Pain killing: the use of a hot saline solution at 40 °C during the whole intervention and the final filling of the abdominal cavity enable the last cc CO2 to go out and almost completely remove post-surgery pains.

In conclusion, from the clinical point of view, the use of a washing system which allows the immediate setting through a display both of the temperature (from ambient to 40 °C), and of the flow-rate, allows the operator to always have the chance of adequately acting in the various steps of the intervention, without useless and risky waits. In the conclusive step, all physiologic solution can be used which is necessary for the sub-aquatic inspection, without lowering the patient's body temperature. The patient already subjected to CO2 blowing at a lower temperature than his body temperature does not risk any hypothermia even during long interventions. The anesthesiologists therefore are facilitated during the whole intervention, and the patient will have a sweeter wake-up and practically absent post-surgery pains.

As regards commercial advantages, the inventive system gives the opportunity to propose a single apparatus which provides for the use of a hot physiologic solution, while the system itself is not a further expense with respect to already used systems, but rather an alternative with not comparable performances which are much greater.

A further feature of the inventive system is that, differently from prior art systems in which liquids are heated, the system of the invention performs a "bain marie" heating, in which the physiologic solution practically passes from the bags in which it has originally been supplied, directly into the patient, guaranteeing a higher sterility safety.

Some preferred embodiments of the present invention have been previously shown and described: numerous variations and modifications, functionally equivalent to the previous ones, will immediately appear to the skilled people in the art, and fall within the scope of the invention as appears from the enclosed claims. For example, the inventive system 1 can be adapted to operate with a dedicated disposable circuit (not shown) with a bar code reading to activate the system 1 itself for four hours, and with steel cannulas having different sizes and which can be re-used.

## Claims

1. Portable system (1) for heating, washing and sucking liquids for laparoscopic and laparotomic surgery, gynecology and urology, comprising:
- at least one case (5) composed of: a first part made as a tank (7) for liquids; and a second part containing at least one peristaltic pump (11) for sending a variable jet of liquid inside abdominal cavities, and at least one valve (13) for sucking liquids for washing the surgery field;
wherein said system (1) further comprises:
- at least one trolley (3) for supporting and handling, said case (5) being placed on said trolley (3) and said tank (7) being equipped with an inclined bottom to enable discharging liquids and cleaning the tank (7);
- at least one container (15) of liquids placed on said trolley (3) and equipped with a double pump (16) for taking and removing liquids into and from said tank (7); and
- at least one device (18) for keeping the temperature of liquids at an established maximum value, said device (18) heating both said container (15) and said tank (7).

2. System (1) according to claim 1, **characterized in that** it is further equipped with a displaying device (20) for setting the operating parameters of the system (1).

3. System (1) according to claim 2, **characterized in that** said displaying device (20) is of the touch-screen type and allows entering operating parameters and commands for the system (1).

4. System (1) according to claim 1, 2 or 3, **characterized in that** it is further equipped with at least one depurating device (21) for liquids in order to keep them clean upon every loading and emptying passage of said tank (7).

5. System (1) according to any one of the previous claims, **characterized in that** said case (5) is equipped with two separate covers (22, 24) for the two parts of which it is composed, said covers (22, 24) providing the chance of having different opening widths, with room for the passage of disposable tubes for liquids, in order to avoid that such tubes are squashed by possible manoeuvres.

6. System (1) according to any one of the previous claims, **characterized in that** said trolley (3) is equipped with a plurality, preferably four, of pivoting wheels (26) capable of being blocked, for its operating handling.

7. System (1) according to any one of the previous claims, **characterized in that** said system (1) is further equipped with a blocking device of the case (5) for preventing its possible fall.

8. System (1) according to any one of the previous claims, **characterized in that** said valve (13) for sucking liquids is of the gate type and is controlled by control means, preferably of the pedal type.

9. System (1) according to any one of the previous claims, **characterized in that** the temperature of liquids is set at a maximum value of 40 °C.

10. System (1) according to any one of the previous claims, **characterized in that** said liquids are composed of heated sterile physiologic water.

## Patentansprüche

1. Tragbares System (1) zur Erhitzung, Wäsche und Absaugung von Flüssigkeiten für laparoskopische und laparotomische Chirurgie, Gynäkologie und Urologie, das Folgendes enthält:
- mindestens eine Hülle (5), die aus Folgendem besteht: ein erster Teil, der wie eine Schale (7) für die Flüssigkeiten ausgeführt wurde; und ein zweiter Teil, der mindestens eine Peristaltikpumpe (11) enthält, um einen variablen Flüssigkeitsstrahl in den Bauchraum zu leiten, und mindestens ein Ventil (13) für die Absaugung der Flüssigkeiten für die Wäsche des Operationsbereiches;
wo das genannte System (1) außerdem Folgendes umfasst:
- mindestens einen Stütz- und Transportwagen (3), die genannte Hülle (5) ist am genannten Wagen (3) angebracht, und die genannte Schale (7) ist mit einem schrägen Boden ausgestattet, um die Entleerung der Flüssigkeiten und die Reinigung der Schale (7) zu vereinfachen;
- mindestens einen Flüssigkeitsbehälter (15), der am genannten Wagen (3) angebracht ist und mit einer Doppelpumpe (16) ausgestattet ist, um die Flüssigkeiten in die und aus der genannten Schale (7) zu leiten bzw. zu entfernen; und
- mindestens eine Vorrichtung (18) für die Beibehaltung der Flüssigkeitstemperatur auf einem festgelegten Höchstwert, die genannte Vorrichtung (18) erhitzt sowohl den genannten Behälter (15) als auch die genannte Schale (7).

2. System (1) gemäß Patentanspruch 1, das **dadurch gekennzeichnet ist, dass** es außerdem mit einer Anzeigevorrichtung (20) ausgestattet ist, um die Betriebsparameter des Systems (1) einzustellen.

3. System (1) gemäß Patentanspruch 2, das **dadurch gekennzeichnet ist, dass** die genannte Anzeigevorrichtung (20) ein Berührungsbildschirm ist und die Eingabe der Parameter und Steuerbefehle für das System (1) ermöglicht.

4. System (1) gemäß Patentanspruch 1, 2 oder 3, das **dadurch gekennzeichnet ist, dass** es außerdem mit einer Reinigungsvorrichtung (21) für die Flüssigkeiten ausgestattet ist, um diese bei jedem Wechsel der Befüllung und Entleerung der genannten Schale (7) sauber zu halten.

5. System (1) gemäß einem beliebigen der vorhergehenden Patentansprüche, das **dadurch gekennzeichnet ist, dass** die genannte Hülle (5) mit zwei Deckeln (22, 24) ausgestattet ist, die in die zwei Teile aufgeteilt sind, aus denen es besteht, die genannten Deckel (22, 24) bieten die Möglichkeit unterschiedlicher Öffnungsweiten, mit Platz für den Durchgang von Einwegleitungen für die Flüssigkeiten, um zu vermeiden, dass diese Leitungen durch etwaige Manöver zerquetscht werden.

6. System (1) gemäß einem beliebigen der vorhergehenden Patentansprüche, das **dadurch gekennzeichnet ist, dass** der genannte Wagen (3) mit mehreren, vorzugsweise vier, drehbaren und blockierbaren Rädern (26) für seine Betriebsbewegung ausgestattet ist.

7. System (1) gemäß einem beliebigen der vorhergehenden Patentansprüche, das **dadurch gekennzeichnet ist, dass** es außerdem mit einer Blockiervorrichtung der Hülle (5) ausgestattet ist, um zu vermeiden, dass sie herunterfällt.

8. System (1) gemäß einem beliebigen der vorhergehenden Patentansprüche, das **dadurch gekennzeichnet ist, dass** das genannte Ventil (13) für die Flüssigkeitsabsaugung ein Absperrschieber ist und durch Steuervorrichtungen gesteuert wird, vorzugsweise mit einem Pedal.

9. System (1) gemäß einem beliebigen der vorhergehenden Patentansprüche, das **dadurch gekennzeichnet ist, dass** die Flüssigkeitstemperatur auf einen Höchstwert von 40 °C eingestellt wird.

10. System (1) gemäß einem beliebigen der vorhergehenden Patentansprüche, das **dadurch gekennzeichnet ist, dass** die genannten Flüssigkeiten aus erhitztem sterilen physiologischen Wasser bestehen.

## Revendications

1. Système portatif (1) de chauffage, de lavage et d'aspiration de liquides pour la chirurgie laparoscopique et par laparotomie, gynécologique et urologique, comprenant :
- au moins un boîtier (5) composé de : une première partie constituée d'une cuve (7) pour les liquides ; et une seconde partie contenant au moins une pompe péristaltique (11) pour envoyer un jet variable de liquide à l'intérieur de cavités abdominales et au moins une vanne (13) pour l'aspiration des liquides pour le lavage de la chambre d'opération ;
où le système (1) comprend aussi :
- au moins un chariot (3) de support et de mise en mouvement, le boîtier (5) se trouve sur le chariot (3) et la cuve (7) est dotée d'un fond incliné pour faciliter l'évacuation des liquides et son nettoyage ;
- au moins un conteneur (15) des liquides placés sur le chariot (3) et doté d'une double pompe (16) pour porter les liquides dans la cuve (7) et les évacuer de cette dernière ; et
- au moins un dispositif (18) pour le maintien de la température des liquides à une valeur maximale établie ; le dispositif (18) chauffe le boîtier (15) et la cuve (7).

2. Système (1), selon la revendication 1, **caractérisé en ce qu'**il est doté aussi d'un dispositif de visualisation (20) pour programmer les paramètres opérationnels du système (1).

3. Système (1), selon la revendication 2, **caractérisé en ce que** le dispositif de visualisation (20) est doté d'un écran tactile qui permet d'insérer les paramètres et les commandes opérationnelles pour le système (1).

4. Système (1), selon la revendication 1, 2 ou 3, **caractérisé en ce qu'**il est doté aussi d'un dispositif épurateur (21) pour les liquides afin de les maintenir propres à chaque passage de remplissage et vidage de la cuve (7).

5. Système (1), selon l'une des revendications précédentes, **caractérisé en ce que** le boîtier (5) est doté de deux couvercles (22, 24) séparés par les deux parties dont il est composé, ces couvercles (22, 24) offrent la possibilité de largeurs d'ouverture différentes, avec l'espace pour le passage de tubes jetables pour les liquides pour éviter que ces derniers soient écrasés durant des manœuvres éventuelles.

6. Système (1), selon l'une des revendications précédentes, **caractérisé en ce que** le chariot (3) est doté d'une pluralité, de préférence quatre, de roues (26) pivotantes et blocables pour sa mise en mouvement opérationnelle.

7. Système (1), selon l'une des revendications précédentes, **caractérisé en ce que** le système (1) est doté aussi d'un dispositif de blocage du boîtier (5) pour empêcher sa chute éventuelle.

8. Système (1), selon l'une des revendications précédentes, **caractérisé en ce que** la vanne (13) d'aspiration des liquides est à passage directe et est commandée de préférence par une pédale.

9. Système (1), selon l'une des revendications précédentes, **caractérisé en ce que** la température des liquides est prédisposée sur une valeur maximale de 40 °C.

10. Système (1), selon l'une des revendications précédentes, **caractérisé en ce que** les liquides sont constitués d'eau physiologique stérile réchauffée.
